# EUROPEAN PATENT SPECIFICATION

(11) **EP 4 380 549 B1**
(45) Date of publication and mention of the grant of the patent: **13.11.2024**
(21) Application number: 22760745.4
(22) Date of filing: 03.08.2022
(51) Int. Cl.: A61K 9/08, A61K 31/00, A61K 47/10, A61K 47/36, A61K 38/48

(54) **PEPTIDASE FORMULATION FOR TREATMENT OF MICROBIAL INFECTIONS IN THE UPPER RESPIRATORY TRACT**
PEPTIDASEFORMULIERUNG ZUR BEHANDLUNG VON MIKROBIELLEN INFEKTIONEN IN DEN OBEREN ATEMWEGEN
FORMULATION DE PEPTIDASE POUR LE TRAITEMENT D'INFECTIONS MICROBIENNES DANS LES VOIES RESPIRATOIRES SUPÉRIEURES

(30) Priority: 03.08.2021 EP 21189429
(43) Date of publication of application: 12.06.2024
(73) Proprietor: ZYMIQ Technology AB, 223 63 Lund (SE)
(72) Inventor: CLARSUND, Mats, 261 93 Saxtorp (SE)
(74) Representative: Høiberg P/S
(86) International application number: PCT/EP2022/071842
(87) International publication number: WO 2023/012224

(56) References cited:
- EP-A1- 3 736 331
- WO-A1-2019/135003
- CN-C- 100 584 376
- US-B1- 11 013 687
- US-B1- 6 599 883

## Description

### Technical field

The present invention relates to a stable peptidase formulation for the treatment and prevention of microbial infections in the upper respiratory tract.

### Background

Upper respiratory tract infections are caused by an acute infection, which involves the upper respiratory tract, including the nose, sinuses, pharynx, or larynx. Upper respiratory tract infection is one of the most common infectious illnesses in the general population, resulting in common cold and influenza, which are major contributors to unwanted absence from work and school. The vast majority of upper respiratory infections are caused by viruses. Over 200 different viruses have been isolated in patients with upper respiratory tract infections such as rhinoviruses, influenza viruses, Respiratory Syncytial Viruses (RSV), coronaviruses, parainfluenza viruses, adenoviruses, enteroviruses, metapneumo-viruses and unknown viruses (Heikkinen et al, 2003).

For SARS-CoV-2 patients, nasal swabs have yielded higher viral loads than throat swab implicating the nasal epithelium as a portal for initial infection and transmission (Sungnak et al, 2020). Given that nasal carriage is likely to be a key feature of transmission of SARS-CoV-2, drugs/vaccines administered intranasally could be highly effective in limiting spread.

Viral infections varies from being relatively mild short-term infections, such as common cold or more severe viral infections such as influenza which spreads worldwide in seasonal epidemics, to aggressive life-threatening infections. In addition to the seasonal epidemics there have been several pandemics, the most devastating being the Spanish Flu of 1918, which caused 20-40 million deaths globally. Nearly half of the influenza-related deaths during the pandemic occurred among young, healthy adults for reasons that remain obscure but are suspected to be related to secondary bacterial infections (Mallia and Johnston, 2007). Bacterial superinfection in viral respiratory disease is a clinically well documented and physical damage to respiratory cells as a result of viral infection may lead to opportunistic adherence of bacteria.

Given the magnitude of the cold flu and influenza health problems, in addition to new emerging viral infections that are difficult to treat such as MERS (Middle East respiratory syndrome) and COVID-19, the need for novel therapeutics against viral infections is evident.

Vaccination is currently the primary focus to prevent spread of influenza virus but due to mutations new vaccines must be developed each year. Development of antiviral drugs against common cold are difficult because of the diversity of viruses responsible for this disease. Such therapeutics should have broad-spectrum activity in order to be suited as an antiviral measure against diverse range of viruses.

Prophylactic pharmaceutical agents that have demonstrated to prevent cold infections exist, however these show side effects, such as intranasal bleeding. Other antiviral chemotherapies (e.g., ICAM-1 blocker, capsid binding agents, and protease inhibitors) have similarly failed to show a promising risk to benefit ratio (Allan et al, 2014).

There is an urgent need to develop new highly stable topical antimicrobial compositions, such as anti-viral compositions with broad spectrum activity (i.e. effective against various enveloped and nonenveloped viruses, RNA and DNA based genome, and independent on different viral proteins (mutated or not)), that reduce and/or prevents virus from binding to cells. Such compositions are ideally based on non-toxic compounds. Further, such anti-viral compositions should also be effective against secondary infections in the upper respiratory tract caused by bacteria and fungi, and target microbial/bacterial adhesion that do not induce resistance and furthermore has no or low impact on patients' natural microbiota.

CN 100 584 376 C discloses a compositions comprising proteinases and sugar alcohols. WO 2019/135003 A1 discloses a nasal sprayable composition comprising trypsin. US 6 599 883 B1 discloses a pharmaceutical composition for preventing and/or treating upper respiratory infections, said composition comprising xylitol as an active reagent. US 11 013 687 B1 discloses a nasal sprayable solution comprising carrageenan and xylitol. EP 3 736 331 A1 discloses a composition comprising trypsin and glycerol in a sprayable solution.

### Summary

The inventors of the present invention have developed a novel formulation. Said formulation is a stable peptidase formulation for prevention and treatment of viral infections in the upper respiratory tract. The formulation can be administrated to the upper respiratory tract by nasal administration, thereby reaching the nasopharynx as well as by oral administration to reach the pharynx.

This disclosure describes a product that functions as a complement to vaccines and systemic antivirals. It forms a thin protective broad-spectrum antiviral gel in the nasal cavity and/or throat (the pharynx) to prevent uptake of virus locally in upper respiratory tract or reduce propagation of viral infections. Virus is trapped and bound to the gel and then deactivated by a broad spectrum peptidase. The formulation constitutes an effective barrier, and exhibits low-to-no stinging when applied in the nasal cavity.

Said formulation is capable of reducing SARS-CoV-2 virus by >99.9% *in vitro.*

The present invention is also active against bacteria and fungi by targeting the same virulence factor as for viruses namely the binding interaction between microbials and cells. Said formulation does not induce microbial resistance and is therefore a good alternative to antibiotics. Thus, the formulation described herein carry high potential for clinical use and provides a solution to the challenge of outphasing the use of antibiotics.

In one aspect, the disclosure relates to a stable composition comprising:
i. peptidase;
ii. sugar alcohol;

In another aspect, the disclosure relates to a composition comprising:
i. peptidase;
ii. glycerol;
iii. xylitol;
iv. hyaluronic acid; and
v. buffer.

In another aspect, the disclosure relates to said composition for use in medicine.

In yet another aspect, the disclosure relates to a composition comprising or consisting essentially of:
i. trypsin;
ii. glycerol;
iii. xylitol;
iv. hyaluronic acid;
v. CaCl₂; and
vi. buffer.

On aspect provides for a composition as disclosed herein for use in the treatment of conditions selected from the group consisting of microbial infections, dermatological conditions, and oral conditions, in and/or on a mammal.

One aspect provides for a composition as disclosed herein for use in the treatment and/or prevention of upper respiratory tract infections, said composition being formulated for nasal and/or oral administration.

In one aspect, the disclosure provides for a method for prevention and/or reduction of microbial infections, wherein the method comprises administration of the composition disclosed herein.

In yet another aspect, the disclosure relates to a method for increasing microbial diversity, wherein the method comprises administration of the composition disclosed herein.

In one aspect, the disclosure relates to a method of manufacturing the composition disclosed herein, said method comprising mixing:
i. glycerol;
ii. xylitol;
iii. buffer;
iv. hyaluronic acid; and
v. peptidase.

The invention is defined by the claims. Any subject matter falling outside the scope of the claims is provided for information purposes only

### Description of Drawings

**Figure 1****.** Enzyme activity as a function of dilution. Highest activity was found at a glycerol concentration of 25-30 wt%.
**Figure 2****.** Effect of increasing Carrageenan concentration on enzyme activity. Carrageenan concentration of >0.2 mg/ml impacts activity.
**Figure 3****.** Enzyme stability in various formulations. Addition of >10% xylitol to 25-30 % glycerol was required in order to achieve high stability. CX is a commercially available, trypsin-containing mouth spray.
**Figure 4****.** Enzyme stability in various formulations. Formulations N2 and N4, without added carrageenan, expressed high stability. CX is a commercially available, trypsin-containing mouth spray.
**Figure 5****.** Instantaneous viscosity for glycerol containing increasing concentrations of carrageenan.
**Figure 6****.** Enzyme stability in TS01, TS02, TS02 without xylitol and CX. CX is a commercially available trypsin-containing mouth spray.

### Detailed description

### Definitions

The term "microbial infection" as used herein refers to an infection with a microorganism, such as a fungus, bacteria or virus. The term "viral infection" refers to any stage of a viral infection, including incubation phase, latent or dormant phase, acute phase, and development and maintenance of immunity towards a virus.

By "upper respiratory tract" the mouth, nose, sinus, middle ear, throat, pharynx, larynx, and trachea are included.

As used herein, terms such as "preventing viral infection" and "treating viral infection" means to inhibit the replication of the particular virus, to inhibit viral transmission, or to prevent the virus from establishing itself in its host, and to ameliorate or alleviate the symptoms of the disease caused by the viral infection, e.g. the sore throat, blocked and/or runny nose, cough and/or elevated temperature. The treatment is considered therapeutic if there is a reduction in viral load, and/or a decrease in morbidity and/or mortality.

The term "peptidase" as used herein includes proteases, proteinases and proteolytic enzymes, functional homologues or functionalised derivatives thereof, capable of cleaving a polypeptide of any length, short or long, at a peptide bond, for example by hydrolysing the peptide bond. The substrate of a peptidase is thus a polypeptide.

The term "sugar alcohol" as used herein refers to a sugar derivative, monosaccharides, disaccharides, oligosaccharides, whose carbonyl group (aldehyde or ketone, reducing sugar) has been reduced to a primary or secondary hydroxyl group. Non-limiting examples of sugar alcohols include: ethylenglycol (2-carbon), glycerol (3-carbon), erythritol (4-carbon), threitol (4-carbon), arabitol (5-carbon), xylitol (5-carbon), ribitol (5-carbon), mannitol (6-carbon), sorbitol (6-carbon), dulcitol (6-carbon), iditol (6-carbon), isomalt (12-carbon), maltitol (12-carbon), lactitol (12-carbon), polyglycitol and other relevant polyol derivatives.

The term "polyol" as used herein refers to a compound comprising multiple alcohol groups, such as propylene glycol. As used herein, polyol does not necessarily have one hydroxy group on each carbon atom.

The term "buffer" as used herein refers to an aqueous solution containing an acid-base mixture with the purpose of stabilizing pH.

The term "hyaluronic acid" (HA) as used herein refers to a polymer comprising repeat disaccharide subunits of hyaluronan. The term also includes a polymer in which the disaccharide subunits may be derivatized at one or more positions of the D-glucuronic acid and/or the D-N-acetylglucosamine repeat subunit. Accordingly the term hyaluronic acid encompasses hyaluronic acid, cross-linked forms, derivatized forms of hyaluronic acid, and pharmacologically acceptable salts thereof.

The term "pharmaceutically acceptable" as used herein generally refers to those compounds, materials, compositions, and/or dosage forms which are, within the scope of sound medical judgment, suitable for use in contact with the tissues, organs, and/or bodily fluids of human beings and animals without excessive toxicity, irritation, allergic response, or other problems or complications commensurate with a reasonable benefit/risk ratio.

The term "stability" as used herein can refer to *in vivo* stability or storage stability (e.g., storage stability of a peptidase at room temperature).

The term "flavouring agent" as used herein refers to any substance, natural or unnatural, that is capable of imparting a detectable flavour impact, especially at a concentration below 4 wt.%, more preferably below 2 wt.%.

The term "carrageenan" as used herein refers to a family of linear sulfated polysaccharides which are usually extracted from red seaweeds. All carrageenans are polysaccharides made up of repeating galactose units and 3,6 anhydrogalactose, both sulfated and non-sulfated. Various types of carrageenans exist. Among them the main types are kappa, lambda and iota. Carrageenan are typically used for viscosity control or for achieving bioadhesion.

The term "bioadhesion" as used herein refers to the ability of certain materials such as polymers, to adhere to biological or body tissue. The term "mucoadhesion" as used herein, refers to the ability of certain materials to adhere, through the formation of chemical and/or physical bonds, to mucosa or mucosal tissue (as e.g. the nasal mucosa).

The term "viscosity" as used herein refers to a fluid's resistance to flow. The unit of viscosity is Pascal-second (Pa.s or Pas).

Terms such as "oral", "throat", and "by oral administration" as used herein refer to the introduction of a pharmaceutical composition into a subject by way of the oral cavity. In a preferred embodiment, the pharmaceutical composition is a liquid composition.

Terms such as "nasal," and "by nasal administration" as used herein refer to the introduction of a pharmaceutical composition into a subject by way of the nasal cavity. In a preferred embodiment, the pharmaceutical composition is a liquid composition.

The term "tolerable" as used herein refers to the irritation, stinging sensation and secretory stimulation observed e.g. when the composition is used for nasal administration. Tolerability is rated from unacceptable to comfortable herein as assessed by the test subject.

As generally used herein, the term "divalent cation" refers to a positively charged ion of any metal from the periodic table having a valence of 2. Where an amount of divalent cation, in an aqueous solution, for example, is given in a percentage concentration, such as % w/w the concentration of divalent cation is based on the weight of the cation in relation to the total weight of the composition of which the cation forms part.

In the context of the present disclosure, the term "w/w" means a "weight/weight". The expression "% w/w" is synonymous to "wt%" or "weight-percent". By way of example, 10 g of a composition comprising 50 % w/w of A comprises 5 g A.

In the context of the present disclosure, phrases such as "a composition comprising X to Y % of A" are taken to mean a composition comprising A in the range of X to Y %, both thresholds included. That is, the composition does not comprise less than X % of A, and the composition does not comprise more than Y % of A, provided X is smaller than Y.

### Nasal anatomy and tolerability

The most common route of viral entry is through the respiratory tract. The respiratory tract refers to the pathway that carries air to the lungs, and can be divided into upper and lower parts. The upper respiratory tract includes the nose, sinuses, throat (pharynx) and voice box (larynx). Mechanical barriers play a significant role in anti-viral defence of the upper respiratory tract. For example, the tract is lined with a mucociliary blanket consisting of ciliated cells, mucous-secreting goblet cells, and sub-epithelial mucous-secreting glands. Foreign particles deposited in the nasal cavity or upper respiratory tract are trapped in mucus, carried to the back of the throat, and swallowed. In the lower respiratory tract, particles trapped in mucus are brought up from the lungs to the throat by ciliary action. The lowest portions of the tract, the alveoli, lack cilia or mucus, but macrophages lining the alveoli ingest and destroy particles. Other cellular and humoral immune responses also intervene. To infect the respiratory tract successfully, viruses must not be swept away by mucus, neutralized by antibody, or destroyed by alveolar macrophages.

One common factor shared by all the common cold viruses, and other infectious viruses of the respiratory tract, is that they must evade the mechanical defence mechanisms of the respiratory tract. Microbial adhesion, where microbial surface proteins recognize cell surface protein, is the most common mode of establishing an infection in the host (Meena et al, 2020).

This invention seeks to strengthen the natural mechanical barrier towards microbial infection. Blocking or hindering migration of virus to reach specific host cell receptors is an attractive way to provide a safe and efficacious protection against virus.

For best broad-spectrum protection against viral infection in upper respiratory tract, both nasal and oral cavity should be protected.

Generally the mucosa of the nasal cavity is more sensitive to hyperosmotic solutions than the oropharynx. Local irritation and nasal burning/pain has been reported after nasal administration of e.g. Nasalide^{®} nasal spray, where up to 45% of patients noted nasal burning (Trangsrud et al, 2002).

The present invention takes both the rapid clearance and the sensitivity of the nasal cavity into account.

### Peptidase

Development of antiviral drugs against common cold viruses is challenging, because of the diversity of viruses responsible for this disease. In the present disclosure, peptidase compositions for prevention of microbial infections are employed. The term "peptidase" as used herein includes proteases, proteinases and proteolytic enzymes and functional homologues or functionalized derivatives thereof, capable of catalyzing proteolysis *in vivo*, in the mammalian (e.g. human) body, thereby cleaving polypeptides of any length, short or long, at a peptide bond, for example by hydrolyzing the peptide bond. The substrate can be a naturally occurring polypeptide or a synthetic polypeptide. The peptidase may be either specific, being capable of hydrolysing only select peptide bonds, or may be non-specific, being capable of hydrolysing many different peptide bonds.

Viruses and bacteria may be present in the nasopharynx without causing any respiratory symptoms. The upper respiratory tract hosts a vast range of commensals and potential pathogenic bacteria, which form a complex microbial community. This community is assumed to be constantly subject to synergistic and competitive interspecies interactions. Disturbances in the equilibrium, for instance due to the acquisition of new bacteria or viruses, may lead to overgrowth and invasion.

Peptidases are very suitable for reduction or inhibition of unwanted, microbial adhesion such as viral adhesion, and furthermore peptidases are non-toxic, well-tolerated, and do not induce resistance. Compositions comprising peptidases can be used as therapeutic agents, as an alternative to antibiotics in treatments against microbial infections without interfering with the desired, healthy microbiota.

### Stable peptidase composition

The present disclosure relates to a stable peptidase composition for prevention and/or treatment of microbial infections. Said composition provides a means to strengthen the intrinsic barrier to increase protection against viruses. The disclosed composition comprises a peptidase, which can hydrolyse the microbial surface proteins thereby preventing microbial adhesion and the establishment of microbial infections. Furthermore the composition comprises a mixture of sugar alcohols or polyols, capable of providing a stable composition, tolerable for nasal administration.

The composition of the present disclosure is more efficient against viruses and has improved shelf-life and activity compared to other known peptidase formulations (e.g. commercially available peptidase formulation). Furthermore the composition is suitable for nasal administration to humans and other mammals (as concluded from disclosed Example 4), which is a significant improvement, compared to other known peptidase formulations, which only are tolerable for oral administration.

Experimental results show that the present composition is highly effective for reduction of microbial infections. In a virucidal efficacy test (results presented in Example 5) it was found that both selected formulations (throat and nasal) are more efficient against SARS-CoV-2 virus than a trypsin-comprising commercially available mouth spray in *in vitro* assays.

One embodiment of the present disclosure provides for a composition comprising
i. 0.005 to 1.0 % w/w peptidase;
ii. 1.0 to 70 % w/w sugar alcohol.

In one embodiment, the composition comprises:
i. 0.005 to 1.0 % w/w peptidase;
ii. 20 to 70 % w/w glycerol; and
iii. 1.0 to 65 % w/w xylitol.

In one embodiment, the composition comprises:
i. 0.005 to 1.0 % w/w peptidase;
ii. 20 to 70 % w/w glycerol; and
iii. 1.0 to 65 % w/w xylitol,
wherein the combination of components of said composition does not exceed 100%.

In one embodiment the composition comprises 0.005 to 1.0 % w/w peptidase, such as 0.01 to 0.075 % w/w peptidase, such as 0.015 to 0.055 % w/w peptidase.

In one embodiment, said peptidase is selected from the group consisting of serine proteases (such as trypsins and chymotrypsins), threonine proteases, cysteine proteases, aspartate proteases, glutamic acid proteases and metalloproteases, functional homologues thereof, and functionalised derivatives thereof. In one embodiment, said peptidase is a single type of peptidase, or a mixture of different types of peptidases.

In one embodiment of the present disclosure, the combination of components of said composition does not exceed 100%.

### Peptidase activity

Peptidase activity can be determined by measuring the product formed during a defined time per given conditions per amount of peptidase. The specific activity of a peptidase is the enzymatic activity per protein concentration. Thus, the total peptidase activity will be determined by specific activity and purity. By way of example, a desired activity can be achieved by using a relatively impure peptidase at a relatively higher concentration, or it can be achieved using a relatively pure peptidase at a relatively lower concentration. The optimal concentration of peptidase to be used can be assessed by measuring the enzyme activity.

### Trypsin

Said peptidase may be a protease such as trypsin, which predominantly cleaves peptide chains at the carboxyl side of the amino acids lysine and arginine.

In one embodiment, the peptidase is a protease. In another embodiment said protease is trypsin. In yet another embodiment, the protease is a functional homologue or a functionalized derivative of trypsin having proteolytic activity.

In one embodiment of the disclosure, the composition comprises 0.005 to 1.0 % w/w protease, such as 0.005 to 1.0 % w/w protease. In a further embodiment of the disclosure, the composition comprises 0.015 to 0.055 % w/w protease. In a specific embodiment of the disclosure, the composition comprises essentially 0.015 to 0.055 % w/w protease, such as 0.015 to 0.055 % w/w protease.

In one embodiment of the disclosure, the composition comprises 0.005 to 1.0 % w/w trypsin, a functional homologue or a functionalized derivative thereof, such as 0.005 to 1.0 % w/w trypsin, a functional homologue or a functionalized derivative thereof.

In a further embodiment of the disclosure, the composition comprises 0.015 to 0.055 % w/w trypsin, a functional homologue or a functionalized derivative thereof. In a specific embodiment of the disclosure, the composition comprises essentially 0.015 to 0.055 % w/w trypsin, a functional homologue or a functionalized derivative thereof, such as 0.015 to 0.055 % w/w.

### Cation such as calcium

The composition of the present disclosure is a stable peptidase formulation. The peptidase may be stabilised by a salt.

In one embodiment the composition further comprises a salt. In another embodiment, said composition comprises a divalent cation.

As used herein, the term "divalent cation" refers to a positively charged ion of any metal from the periodic table having a valence of 2, such as calcium.

In one embodiment, the invention discloses a composition comprising trypsin, said composition further comprising a salt. In another embodiment, the invention discloses a composition comprising trypsin, said composition further comprising a salt with a divalent cation.

In a more specific embodiment, the invention discloses a composition comprising a peptidase, said composition further comprising a salt with a divalent cation, such as 0.003 to 0.2 % w/w salt comprising a divalent cation, such as 0.003 to 0.15 % w/w, such as 0.003 to 0.1 % w/w salt comprising a divalent cation.

In another more specific embodiment, the composition comprising trypsin, a functional homologue, or a functionalized derivative thereof, further comprises 0.003 to 0.2 % w/w salt comprising a divalent cation, such as 0.003 to 0.15 % w/w, such as 0.003 to 0.1 % w/w salt comprising a divalent cation.

In one embodiment of the disclosure, the divalent cation is calcium (Ca²⁺). In another embodiment, the salt comprising the divalent cation is a pharmaceutically acceptable salt of calcium, including hydrates.

In another embodiment the pharmaceutically acceptable salt of calcium is CaCl₂. In yet another embodiment, the salt comprising a divalent cation is a pharmaceutically acceptable inorganic salt of calcium, including hydrates thereof, such as calcium chloride dihydrate. In another embodiment, the salt comprising a divalent cation is a pharmaceutically acceptable organic salt of calcium, such as calcium acetate or calcium citrate.

In one embodiment the composition comprises 0.003 to 0.2 % w/w calcium, such as 0.003 to 0.15 % w/w, such as 0.003 to 0.1 %.

In one embodiment the composition further comprises calcium ions, such as 0.003 to 0.2 % w/w calcium ions, such as 0.003 to 0.15 % w/w calcium ions, such as 0.003 to 0.1 % w/w calcium ions. Calcium ions means Ca²⁺.

In another embodiment the composition comprises 0.002 to 0.2 % w/w calcium chloride, such as 0.005 to 0.02 % w/w calcium chloride, such as 0.010 to 0.015 % w/w calcium chloride.

In yet another embodiment the composition comprises 0.002 to 0.2 % w/w calcium chloride dihydrate, such as 0.005 to 0.02 % w/w calcium chloride dihydrate, such as 0.010 to 0.015 % w/w calcium chloride dihydrate.

In one embodiment of the disclosed, the composition comprises:
i. 0.005 to 1.0 % w/w trypsin;
ii. 1.0 to 70 % w/w sugar alcohol; and
iii. 0.010 to 0.015 % w/w calcium dichloride dihydrate.

### Sugar alcohol/polyol

The composition of the present disclosure is a stable peptidase formulation. Deactivation of the peptidase improves its stability in the composition. One way to deactivate the peptidase is by reducing the water activity of the composition. Water activity of an aqueous solution can be reduced by presence of ions and/or other molecules.

The present invention comprises salts, sugar alcohols, and/or polyols, which in part act to reduce the water activity in the composition. Reducing the water activity in turn deactivates the peptidase. Furthermore it is beneficial for the purpose of the present disclosure that said composition is hyperosmotic. Hyperosmotic compositions generally stimulate nasal secretion, which has a rinsing effect of the nasal mucosa cleaning the infected surface of all the contaminants, such as virus particles and free-floating bacteria, without any toxic effect on the cells or the cellular matrix.

In one embodiment of the present disclosure, the composition comprises one or more of a diol, a triol, or a polyol, such as glycerol, propylene glycol, or a sugar alcohol.

In another embodiment, the composition comprises 35 to 70 % w/w sugar alcohol, such as 40 to 65 % w/w sugar alcohol, such as 45 to 60 % w/w sugar alcohol.

The sugar alcohol may be glycerol, erythritol, xylitol, mannitol, arabitol, maltitol, lactitol, isomalt, sorbitol, hydrogenated starch hydrolysates or propylene glycol and any other suitable polyol or mixtures thereof providing a stable solution of the peptidase.

The oral cavity tolerates high concentrations of glycerol well whereas high concentrations of glycerol has a painful, stinging effect on the thinner, more sensitive nasal mucosa. A composition comprising 50% glycerol cannot be sprayed into nasal cavity because it stings severely due to the high hypertonic effect of glycerol. 10-25% glycerol can be accepted at low volumes. However, at low glycerol concentration after dilution (25%), the enzymes disclosed herein are not stable for long term storage, severely reducing their use in treatment and/or prevention of infections.

As the nasal mucosa is more sensitive to hyperosmotic solutions than the oropharynx, the glycerol concentration in said formulation for nasal use, needs to be diluted compared to formulations for oral use, while maintaining a stable composition.

In an effort to minimize irritation of the nasal passage, various combinations and concentrations of sugars and sugar alcohols were screened for their applicability, and it was surprisingly found that the disclosed composition, comprising a mixture of the sugar alcohols glycerol and xylitol, provides a stable composition, said stable composition being tolerable for nasal administration.

Xylitol is a sugar alcohol with properties similar to glycerol. Xylitol can have a protein stabilizing effect. Xylitol furthermore has a sweetness intensity equal to sucrose.

In one embodiment the composition comprises 20 to 70 % w/w glycerol, such as 25 to 65 % w/w glycerol. In one specific embodiment, the composition comprises 27 to 57 % w/w glycerol.

In another embodiment the composition comprises 1.0 to 65 % w/w xylitol, such as 3.0 to 50 % w/w xylitol, such as 4.5 to 30 % w/w xylitol.

In yet another embodiment the composition comprises a combined amount of glycerol and xylitol of at least 40 % w/w, such as at least 45 % w/w, such as at least 50 % w/w.

In a preferred embodiment the composition of the present invention comprises:
i. 0.005 to 1.0 % w/w peptidase;
ii. 20 to 70 % w/w glycerol; and
iii. 1.0 to 65 % w/w xylitol.

For the compliance of the patients taking a nasal spray, the taste and tolerance of the product is of particular importance. This invention presents a tolerable formulation with a sweet, pleasant taste.

### Buffer

The term "buffer" is intended to mean an aqueous solution containing an acid-base mixture with the purpose of stabilising pH. pH is of importance for both the stability of the peptidase, the activity of the peptidase, and the tolerability of the composition when used for treatment of microbial infections in the oral and the nasal cavity.

Examples of buffers are Trizma, Bicine, Tricine, MOPS, MOPSO, MOBS, Tris, Hepes, HEPBS, MES, phosphate, carbonate, acetate, citrate, glycolate, lactate, borate, ACES, ADA, tartrate, AMP, AMPD, AMPSO, BES, CABS, cacodylate, CHES, DIPSO, EPPS, ethanolamine, glycine, HEPPSO, imidazole, imidazolelactic acid, PIPES, SSC, SSPE, POPSO, TAPS, TABS, TAPSO and TES. The buffer may be a mixture of two or more buffer systems. In one embodiment, the buffer is any buffer above, including mixtures of two or more buffers. In one embodiment the disclosure, the composition comprises a buffer stabilizing the pH of the composition. The buffer may be selected from the group consisting of tris(hydroxymethyl)amino methane (Tris), 3-(*N*-morpholino)propane sulfonic acid (MOPS), phosphate, or any other suitable buffer, such as pharmaceutically acceptable buffers.

In one embodiment, the composition of the present disclosure comprises 0.01 to 2.4 % w/w buffer, such as 0.07 to 0.3 % w/w buffer, such as 0.09 to 0.2 % w/w buffer.

In another embodiment, the composition comprises a concentration of buffer in the range 1 to 200 mM, such as 5 to 25 mM, such as 8 to 15 mM.

The pH of the composition is adjusted to be suitable for use in respectively the nasal and the oral cavity. The nasal cavity has a pH range of 5.5-6.5. The pH of the throat has a range 7.2 to 8.5. The composition has a pH in the range 5.5 to 8.5, such as 6.0 to 7.0, such as 6.3 to 6.7.

In one embodiment, the composition has a pH in the range 6.2 to 7.0, such as 6.4 to 6.6. In another embodiment, the composition has a pH in the range 7.2 to 8.5, such as 7.4 to 7.6.

The pH of solutions can be assessed in a number of ways. For example, pH can be assessed using a pH electrode or a pH indicator.

In a preferred embodiment, the composition comprises:
i. 0.005 to 1.0 % w/w peptidase;
ii. 20 to 70 % w/w glycerol;
iii. 1.0 to 65 % w/w xylitol; and
iv. 0.01 to 1.2 % w/w buffer.

In one embodiment, the composition further comprises water, such as at the most 50 % w/w water, such as at most 40 % w/w, such as at most 30 % w/w.

### Polymers

For mucosal application, the composition may advantageously comprise ingredients giving mucoadhesive properties to the composition.

The disclosed composition may comprise polymers to increase the residence time of the composition (e.g. gel). In certain embodiments the polymer(s), relative amounts, and concentrations are selected to provide a film that is mucoadhesive. Mucoadhesive performance is typically observed for polymers possessing charged groups or nonionic functional groups capable of forming hydrogen bonds. Negatively charged polysaccharides such as hyaluronate salts of hyaluronic acid, and sulfated polysaccharides such as carrageenan are found to be efficient.

In one embodiment of the present disclosure the composition comprises polymers capable of increasing the residence time of the composition in mucosa. In one embodiment the composition comprises a polymer selected from the group consisting of hyaluronic acid and hyaluronic acid derivatives. Such function as a means to strengthen the gel, increase mucoadhesion and to reduce irritation potential in the nasal cavity.

In one embodiment, polymers are included to strengthen the composition by forming a gel, to reduce irritation potential in the nasal cavity.

### Hyaluronic acid

In one embodiment of the disclosure, the composition comprises hyaluronic acid.

In one embodiment, the composition comprises hyaluronic acid, such as 0.0001 to 2.0 % w/w hyaluronic acid, such as 0.001 to 1.5 % w/w, such as 0.01 to 1.0 % w/w, such as 0.02 to 0.5 % w/w hyaluronic acid.

In one embodiment of the disclosure the composition comprises or consists essentially of:
i. 0.005 to 1.0 % w/w peptidase;
ii. 20 to 70 % w/w glycerol;
iii. 1.0 to 65 % w/w xylitol;
iv. 0.0001 to 1.5 % w/w hyaluronic acid; and
v. 0.01 to 1.2 % w/w buffer.

In another embodiment of the disclosure, the composition comprises or consists essentially of:
i. 0.005 to 1.0 % w/w trypsin;
ii. 20 to 70 % w/w glycerol;
iii. 1.0 to 65 % w/w xylitol;
iv. 0.0001 to 1.5 % w/w hyaluronic acid;
v. 0.002 to 0.2 % w/w CaCl₂; and
vi. 0.01 to 1.2 % w/w buffer.

In another embodiment, the composition comprises or consists essentially of:
i. 0.015 to 0.055 % w/w trypsin;
ii. 27 to 57 % w/w glycerol;
iii. 4.5 to 30 % w/w xylitol;
iv. 0.0002 to 0.02 % w/w hyaluronic acid;
v. 0.010 to 0.015 % w/w CaCl₂; and
vi. 0.1 to 0.14 % w/w Tris buffer.

In one embodiment, the pH of said composition is between 5.5 to 8.5, such as 6.0 to 7.0, such as 6.3 to 6.7. In one embodiment, the pH of said composition is between 6.2 to 7.0, such as 6.4 to 6.6. In one embodiment, the pH of said composition is between 7.2 to 8.5, such as 7.4 to 7.6.

In one embodiment, the composition comprises water. In a specific embodiment of the disclosure, at least part of the water of the composition originates from the buffer. The presence of free water can activate and/or destabilize the peptidase. Water in the composition may be deactivated by other components, such as for instance glycerol and other alcohols describes herein. Accordingly, in one embodiment, the composition comprises at most 50 % w/w water, such as at most 40 % w/w, such as at most 30 % w/w water. To improve the stability of the peptidase, it is important that the water activity in the composition is low. Upon applying the composition to e.g. the nasal cavity, the oral cavity, a wound, or on the skin, water from said nasal cavity, oral cavity, wound or skin mixes with the composition, increasing the water activity. This in turn activates the peptidase.

In another embodiment, the composition is the form of a spray, a lozenge, a pastille, a chewing gum, a gel, or a liquid.

### Carrageenan

In one embodiment the composition comprises carrageenan.

Carrageenan is typically used for viscosity control or for achieving bioadhesion. The term "carrageenan" refers to a family of linear sulfated polysaccharides which are usually extracted from red seaweeds. Carrageenans are polysaccharides made up of repeating galactose units and 3,6 anhydrogalactose, both sulfated and non-sulfated.

In one embodiment the composition comprises no more than 1 % w/w carrageenan, such as no more than 0.5 % w/w carrageenan, such as no more than 0.1 % w/w carrageenan.

### Flavouring agent

For the compliance of the patients being administered the disclosed composition, the smell and taste of the product is of particular importance. This invention presents a tolerable formulation with a sweet pleasant taste in part due to the presence of xylitol. The smell and taste may be further improved, if the composition further comprises a flavouring agent.

The term "flavouring agent" refers to any substance, natural or unnatural, that is capable of imparting a detectable flavour impact, especially at a concentration below 4 % w/w, more preferably below 2 % w/w. Suitable flavours or flavouring agents include, but are not limited to, eucalyptus, mint, such as peppermint and spearmint, menthol, chocolate, liquorice, citrus and other fruit flavours, gamma octalactone, vanillin, ethyl vanillin, breath freshener flavours, spice flavours such as cinnamon, methyl salicylate, linalool, bergamot oil, geranium oil, lemon oil and ginger oil.

In one embodiment, the composition comprises a flavouring agent.

In another embodiment the flavouring agent is natural or unnatural, e.g. synthetic.

In yet another embodiment the natural or unnatural flavouring agent is spearmint or eucalyptus.

In a further embodiment, the composition comprises 0.01 to 0.4 % w/w flavouring agent, such as 0.02 to 0.1 % w/w flavouring agent, such as 0.035 to 0.055 % w/w flavouring agent compound.

In one embodiment, the composition comprises or consists essentially of:
i. 0.02 to 0.05 % w/w peptidase;
ii. 29 to 56 % w/w glycerol;
iii. 4 to 25 % w/w xylitol;
iv. 0.0002 to 0.0004 % w/w hyaluronic acid;
v. 0.1 to 0.2 % w/w buffer;
vi. 0 to 0.1 % w/w carrageenan; and
vii. 0 to 0.05 % w/w flavouring agent.

### Formulation

The disclosure of the present invention is a stable composition for treatment of microbial infections, in particular viral infections in the upper respiratory tract. Virus replication takes place in the upper respiratory tract. Currently developed oral sprays reach the oropharynx while the nasal cavity including the nasopharynx is not reached with the oral administration route. Hence it is highly desired to develop a sprayable composition, which can be administered through the nose to reach the nasal cavity and the nasopharynx, using a nasal spray. For a composition to be sprayable, the composition preferably has a viscosity which is at most 0.015 Pa.s. For the composition to be active against microbial infection in the upper respiratory tract, administration to the upper respiratory tract is required.

In one embodiment, the composition formulated as a spray, lozenge, pastille, chewing gum, gel, or liquid.

In one embodiment, the composition is a sprayable composition.

In another embodiment, the composition is in the form of a spray. In a preferred embodiment, the composition is a nasal spray or a throat spray.

For the composition to be administered using a nasal spray or a throat spray, the viscosity of the composition must allow for the composition to be sprayable.

In one embodiment, the composition has a viscosity which is at most 0.015 Pa.s. This is supported by the findings of Example 6 disclosed herein.

In comparison to an oral spray, the nasal spray targets the nasal cavity, the primary site for upper respiratory tract infections.

### Medical use

The disclosure of the present invention is a stable composition for treatment of microbial infections, in particular viral infections in the upper respiratory tract.

The composition of the present disclosure can complement vaccines and systemic antivirals. It forms a thin protective broad-spectrum antiviral gel in nasal cavity and throat to prevent or reduce propagation of viral infections. Virus is trapped and bound to the gel and then deactivated by a broad spectrum peptidase. In comparison with antiviral drugs that works systemically and within cells to inhibit replication systems, the disclosed composition reduces and/or prevents up-take of virus locally in upper respiratory tract. These mechanisms of action complement each other to achieve more efficient treatment and/prevent of infections. The present invention is also active against bacteria and fungi by targeting the same virulence factor as for viruses namely the proteins responsible for the adhesion between the microbials and the cells.

In one embodiment, the virus infection is selected form the group consisting of common cold, pneumonia, bronchitis, severe acute respiratory syndrome (SARS), Middle East respiratory syndrome (MERS), sinusitis, otitis and pharyngitis. One embodiment provides a method for treatment and/or prevention of virus infection in a mammal comprising administering to the subject a therapeutically-effective amount of a polypeptide having peptidase activity.

The common cold is a mild acute respiratory infection characterized by sore throat, malaise, rhinorrhea, nasal congestion, headache, cough, sneezing, and sometimes mild fever (Jackson et al, 1958). Influenza, is a more severe viral infection, which spreads worldwide in seasonal epidemics infecting up to 20% of the population which depending on the circulating virus, can cause substantial mortality.

In one embodiment, the composition of the disclosure is for use in medicine.

In one embodiment, the disclosure relates to a composition for use in treating and/or preventing of microbial infections.

In one embodiment, the disclosure relates to a composition for use in the treating and/or preventing of a viral infection, a bacterial infection, a fungal infection, and/or a yeast infection.

In one embodiment, the disclosure relates to a composition for use in treating and/or preventing a viral infection.

In one embodiment, the disclosure relates to a composition for use in treating and/or preventing a viral infection from a virus selected from the group consisting of rhinovirus, influenza virus such as influenza A virus, Respiratory Syncytial Virus (RSV), coronavirus, parainfluenza virus, adenovirus, enterovirus, metapneumovirus, and other infectious viruses.

In one embodiment, the disclosure relates to a composition, for use in treating and/or preventing infectious diseases such as common cold, flu, rhinitis, sinusitis, bronchitis, Severe acute respiratory syndrome (SARS), Middle East respiratory syndrome (MERS), coronavirus disease 2019 (COVID-19), Pneumonia, Viral Meningitis, Herpangina, herpesvirus, papillomavirus or any other disease caused by viral infections.

In one embodiment, the disclosure relates to a composition, for use in treating and/or preventing an upper respiratory tract viral infection.

In one embodiment, the disclosure relates to a composition, for use in treating and/or preventing an upper respiratory tract viral infection resulting in common cold, flu, rhinitis, sinusitis, COVID-19, or other diseases caused by upper respiratory tract viral infections.

In one embodiment, the composition as disclosed herein is for use in the prevention and/or treatment of symptoms related to viral infection, such as sore throat, malaise, rhinorrhea, nasal congestion, headache, cough, sneezing, and/or fever.

In one embodiment, the composition as disclosed herein is for use in the prevention and/or treatment of a disease, disorder or condition selected from the group consisting of microbial infections and/or oral conditions.

In one embodiment, the composition as disclosed herein is for use in the prevention and/or treatment of microbial infection selected from the group consisting of viral infections, bacterial infections, fungal infections, and yeast infections.

In another embodiment, the composition as disclosed herein is for use in the prevention and/or treatment of a microbial infection and/or oral condition such as a gum disease in and/or on a mammal.

In another embodiment, the composition as disclosed herein is for use in the prevention and/or treatment of a microbial infection and/or oral condition, such as gingivitis in and/or on a mammal.

In one embodiment, the disclosure provides a composition comprising
i. peptidase;
ii. glycerol;
iii. xylitol;
iv. hyaluronic acid; and
v. buffer
for use in medicine. In one embodiment, the peptidase of said composition is trypsin. In one embodiment, the composition comprising trypsin further comprises a divalent cation such as calcium (Ca²⁺).

One embodiment provides for a composition comprising
i. peptidase;
ii. glycerol;
iii. xylitol;
iv. hyaluronic acid; and
v. buffer
for use in the prevention and/or treatment of a disease, disorder or condition selected from the group consisting of microbial infections and/or oral conditions

In one embodiment the microbial infection and/or oral condition is gingivitis or a gum disease in and/or on a mammal.

One embodiment provides for a composition comprising:
i. 0.005 to 1.0 % w/w peptidase;
ii. 20 to 70 % w/w glycerol;
iii. 1.0 to 65 % w/w xylitol;
iv. 0.0001 to 1.5 % w/w hyaluronic acid; and
v. 0.01 to 1.2 % w/w buffer
for use in the prevention and/or treatment of a disease, disorder or condition selected from the group consisting of microbial infections, and oral conditions.

One embodiment provides for a composition comprising:
i. 0.015 to 0.055 % w/w trypsin;
ii. 27 to 57 % w/w glycerol;
iii. 4.5 to 30 % w/w xylitol;
iv. 0.0002 to 0.02 % w/w hyaluronic acid;
v. 0.010 to 0.015 % w/w CaCl₂; and
vi. 0.1 to 0.14 % w/w buffer
for use in the prevention and/or treatment of a disease, disorder or condition selected from the group consisting of microbial infections, and oral conditions.

One embodiment provides for a composition as disclosed herein, for use in preventing and/or treating a microbial infection selected from the group consisting of viral infections, bacterial infections, fungal infections, and yeast infections.

One embodiment provides for a composition as disclosed herein for use in preventing and/or treating a microbial infection, which is a viral infection. In another embodiment, the composition is for use in the treatment of an upper respiratory tract viral infection. In another more specific embodiment, the microbial infection is an upper respiratory tract viral infections.

One embodiment provides for a composition as disclosed herein for use in in preventing and/or treating an upper respiratory tract infection resulting in common cold, flu, rhinitis, sinusitis, COVID-19, or another disease caused by upper respiratory tract infections.

One embodiment provides for a composition as disclosed herein for use in in preventing and/or treating a viral infection from a virus selected from the group consisting of Rhinovirus, Influenza virus, Respiratory Syncytial Virus (RSV), Coronavirus, Parainfluenza virus, Adenovirus, Enterovirus, Metapneumovirus, and other infectious viruses.

In one embodiment other infectious are other viruses which are capable of infecting and/or entering a cell, is viruses such as Corynebacterium diphtheria, Lassa fever virus (Arenavirus), Astrovirus, Hantavirus, Rift Valley Fever virus (Phlebovirus), Calicivirus, Ebola virus, Marburg Virus, Japanese encephalitis virus, Dengue virus, Yellow fever virus, Hepatitis C virus, Hepatitis G virus, Hepatitis B virus, Hepatitis D virus, Herpes simplex virus, Herpes simplex virus), Cytomegalovirus, Epstein Barr virus, Varicella Zoster Virus, Human Herpesvirus, Human Herpesvirus, Rubella virus, Mumps virus, Morbillivirus, Measles virus, Papillomaviruses, JC virus (Polyomavirus), BK virus (Polyomavirus), Parvovirus, Coxsackie virus (A and B), Hepatitis A virus, Polioviruses, Reovirus, Rabies Virus (Lyssavirus), Human Immunodeficiency virus 1 and 2, Human T-cell Leukemia virus.

One embodiment provides for a composition as disclosed herein for use in in preventing and/or treating a viral infection causing infectious diseases such as common cold, flu, rhinitis, sinusitis, bronchitis, Severe acute respiratory syndrome (SARS), Middle East respiratory syndrome (MERS), coronavirus disease 2019 (COVID-19), Pneumonia, Viral Meningitis, Herpangina, herpesvirus, papillomavirus or any other disease caused by viral infections.

One embodiment provides for a composition as disclosed herein for use in the in preventing and/or treating symptoms related to viral infection, such as sore throat, malaise, rhinorrhea, nasal congestion, headache, cough, sneezing, and/or fever.

One embodiment provides for a composition as disclosed herein for use in the manufacture of a medicament for in preventing and/or treating a disease, disorder or condition selected from the group consisting of microbial infections, dermatological conditions and oral conditions.

In one embodiment the composition is for oral use. In another embodiment, the composition is for nasal use.

In one embodiment, the composition for nasal use has a pH in the range 6.2 to 7.0, such as 6.4 to 6.6. In another embodiment, the composition for oral use has a pH in the range 7.2 to 8.5, such as 7.4 to 7.6.

One embodiment provides for a method of treating or preventing of a disease, disorder or condition selected from the group consisting of microbial infections, dermatological conditions, and oral conditions, said method comprising administering the composition disclosed herein to an individual in need thereof.

The references to methods of treatment in this description are to be interpreted as references to the compounds, pharmaceutical compositions and medicaments of the present invention for use in a method for the treatment of the human (or animal) body by therapy (or for diagnosis)

One embodiment provides for a method of treating or preventing of a disease, disorder or condition selected from the group consisting of microbial infections, dermatological conditions, and oral conditions, said method comprising administration of a composition comprising:
i. peptidase;
ii. glycerol;
iii. xylitol;
iv. hyaluronic acid; and
v. buffer.

In one embodiment, the peptidase of said composition is trypsin. In one embodiment, the composition comprising trypsin further comprises a divalent cation, such as calcium (Ca²⁺).

One embodiment provides for a method of preventing and/or reducing viral infections, said method comprising administering the composition disclosed herein to a subject in need thereof.

One embodiment provides for a method of increasing microbial diversity, said method comprising administering a composition is disclosed herein to a subject in need thereof.

One embodiment provides for a method of manufacturing the composition disclosed herein, said method comprising mixing;
i. sugar alcohol
ii. buffer;
iii. hyaluronic acid; and
iv. peptidase.

One further embodiment provides for a method of manufacturing the composition disclosed herein, further comprising mixing;
i. carrageenan; and/or
ii. a flavouring agent.

One embodiment provides for a method of increasing microbial diversity, said method comprising administering the composition disclosed herein.

### Examples

### Example 1: Enzyme activity as function of dilution in Tris-HCl buffer

The purpose of the experiments was to measure enzyme activity as a function of dilution in Tris-HCl buffer. Further, the carrageenan concentration on enzyme activity was evaluated.

### Methods

7 different formulations with glycerol concentrations from 40 - 15 wt% were prepared, along with formulations with increasing Carrageenan concentration. Enzyme activity was measured. Samples were pulled at predetermined time points and analysed directly by measuring the enzyme activity. The activity was measured at 30°C using Z-Gly-Pro-Arg-pNA as substrate in a spectrophotometer at 405 nm. The decrease in absorbance per minute was used as activity parameter. The remaining activity relative to time point zero was determined and used to describe the stability.

### Results and conclusions

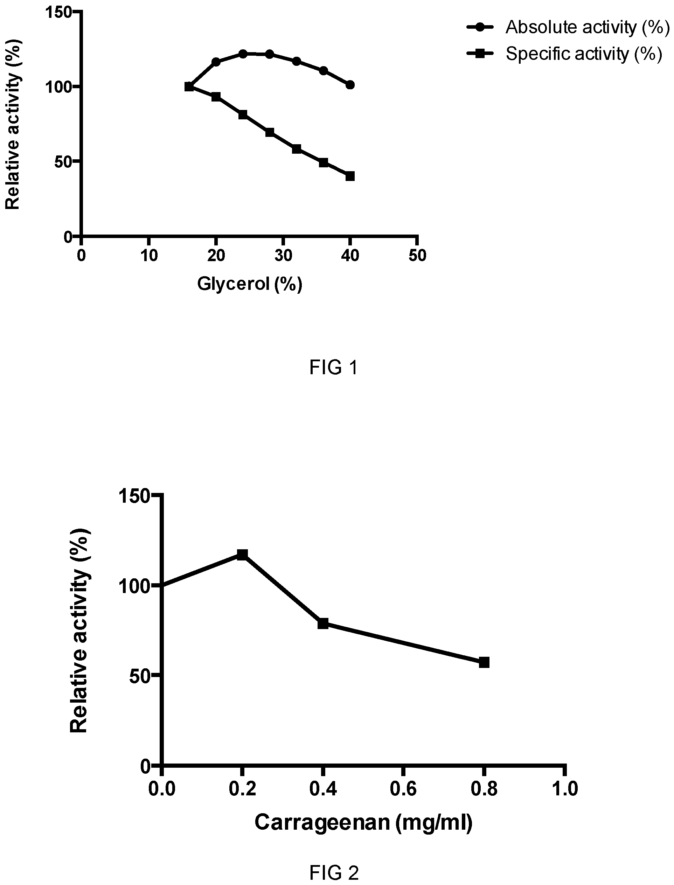

Results from glycerol dilutions are presented in Figure 1. The enzyme activity increases upon dilution with buffer. Maximum absolute activity was obtained at 25-30% glycerol.

The results for formulations with increasing concentrations of added Carrageenan (0-0.8 mg/ml) are presented in Figure 2. A concentration of >0.2 mg/ml seems to reduce activity.

### Example 2: Stability of nasal spray formulations

The purpose of the experiment was to measure enzyme stability in various nasal spray formulations.

### Methods

Various combinations were mixed and the pH was adjusted using Tris buffer to pH 6.5 The formulations described in *Table 1* were tested.

**Table 1: Formulations used in stability studies of nasal sprays. N01-N04 contained CaCl₂ 1 mM, trypsin 0.2 mg/ml, hyaluronic acid 2 µg/ml, Carrageenan 0.5 mg/ml. N05 contained CaCl₂ 1 mM, trypsin 0.2 mg/ml, hyaluronic acid 2 µg/ml.**

| **No** | **Glycerol (wt%)** | **Xylitol (wt%)** |
|---|---|---|
| **N01** | 30 | 0 |
| **N02** | 30 | 10 |
| **N03** | 30 | 25 |
| **N04** | 25 | 25 |
| **N05** | 30 | 25 |

The formulations were put into sealed polypropylene bottles. The stability of products from each formulation composition were tested by placing them at a stressed condition (40 ± 2°C/100% RH) for up to 115 days. Enzyme activity was measured using same method as described in Example 1.

### Results and conclusions

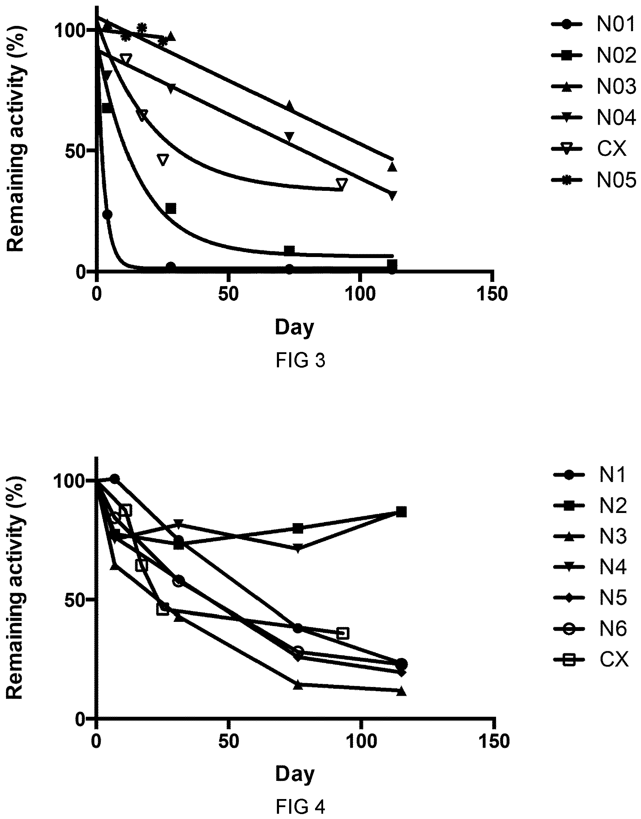

Results are presented in Figure 3. Addition of > 10% xylitol to 25-30 % glycerol resulted in high stability, exemplified by formulations NO3, NO4 & NO5.

These findings indicate the possibility of substituting parts of the glycerol for xylitol to improve stability of the enzyme at stressed conditions of 40 °C. The expected shelf-life for a formulation containing 30 % glycerol (formulation NO1) is < 3-6 months at 25 °C, whereas the expected shelf life of NO5 is expected to be more than 2 years. The presence of excessive amounts of carrageenan decreased the stability.

### Example 3: Stability of throat spray formulations

The purpose of the experiment was to measure enzyme stability in various throat spray formulations.

### Methods

Various formulations, as described in Table 2, were tested. The pH was adjusted to pH 7.5 using Tris buffer.

**Table 2: Formulations used in stability studies of throat sprays.**

| **No** | **Carrageenan (mg/ml)** | **Hyaluronic acid (µg/ml)** | **Xylitol (wt%)** | **Flavour** |
|---|---|---|---|---|
| **N1** | 0.5 | 2 | 5 | |
| **N2** | 0 | 2 | 0 | |
| **N3** | 0.5 | 0 | 0 | |
| **N4** | 0 | 0 | 5 | |
| **N5** | 0.5 | 2 | 5 | Spearmint, 0.05% |
| **N6** | 0.5 | 2 | 5 | Eucalyptus, 0.05% |

All contained 55 wt% glycerol, 1 mM CaCl₂, trypsin 0.45 mg/ml.

The formulations were stored in sealed polypropylene bottles. The stability of products from each formulation composition were tested by placing them at under stressed conditions (40 ± 2°C/100% RH) for up to 115 days. Enzyme activity was measured using same method as described in Example 1

### Results and conclusion

Results are presented in Figure 4. The formulations N2 & N4 expressed the highest stability. Addition of 0.5 % Carrageenan reduced stability, while presence of hyaluronic acid and xylitol showed no negative effect on the stability.

### Example 4: Tolerability of nasal spray formulations

The purpose of the experiment was to evaluate tolerability, irritation/stinging sensation and experienced secretory stimulation of various nasal spray formulations by spraying the formulation into the nasal cavity.

The nasal spray is intended to be hyperosmotic, thereby stimulating nasal secretion, which has a rinsing effect of the nasal mucosa. In an effort to minimize irritation of the nasal passage, based on the observation that the nasal mucosa is more sensitive to hyperosmotic solutions than the oral mucosa, the glycerol concentration in the formulation for nasal use were diluted compared to formulations for oral use.

### Method

The formulations described in Table 3 were prepared and tested.

**Table 3. Formulations used in tolerability studies of throat sprays.**

| **Formulation** | **Glycerol (wt%)** | **Xylitol (wt%)** | **Comments** |
|---|---|---|---|
| **1** | 30 | 5 | * |
| **2** | 30 | 10 | * |
| **3** | 30 | 20 | * |
| **4** | 30 | 25 | * |
| **5** | 30 | 25 | ** |
| **6** | 54 | 0 | * |
| **CX** | 54 | 0 | *** |

| | | | |
|---|---|---|---|
| **Formulation 1-4 and 6 contained glycerol, xylitol, water. **Formulation 5 contained glycerol, xylitol, Tris-HCl, Hyaluronic acid, CaCl₂ and trypsin (concentrations equivalent to N03, Example 2).* *** *CX is a commercially available trypsin-comprising mouth spray.* | | | |

50 µl solution was sprayed, using a nasal spray, in nasal cavity in right and left nostril of two healthy volunteers.

Tolerability was determined immediately after spraying using a 4 graded scale:
0; unacceptable, 1; uncomfortable, 2; acceptable, 3; comfortable.

Secretory stimulation was determined after 2 minutes using a 3 graded scale:
0; no secretion, 1; secretion, 2; runny nose.

Spraying in the nose was repeated 2 times and tolerability and secretory stimulation was noted after each spray.

### Results and conclusion

The average results are presented in Table 4. Both formulation 6 and CX, having a glycerol concentration of 54 wt%, stings severely when sprayed in nasal cavity and causes severe runny nose. Decreasing the glycerol concentration to 30 wt% and adding xylitol in increasing concentration, from 5 to 25 wt% (Formulations 1 to 5), completely eliminated the stinging sensation and tolerability was experienced as acceptable to comfortable. Furthermore, slight secretory stimulation was observed for formulations 4 and 5.

These findings show that it is possible to increase xylitol concentration to 25 wt% in glycerol 30 wt% solution without increasing the stinging sensation when spraying such solution in nasal cavity. This supports that a glycerol/xylitol based formulation is suitable for delivery of trypsin to nasal cavity, as trypsin is stabilised in such solution (Example 2).

**Table 4 Tolerability of nasal spray formulations.**

| **Formulation** | **Tolerability** | **Secretory stimulation** |
|---|---|---|
| 1 | 3 | 0 |
| 2 | 3 | 0 |
| 3 | 3 | 0 |
| 4 | 2,3 | 1 |
| 5 | 2,7 | 0,5 |
| 6 | 0 | 2 |
| CX* | 0 | 2 |

| | | |
|---|---|---|
| **One volunteer.* | | |

### Example 5: Virucidal efficacy of throat spray and nasal spray formulations

The purpose of the experiment was to determine virucidal efficacy of a throat spray formulation (for oral use) and a nasal spray formulation against SARS-CoV-2 virus. In contrast to a throat spray, a nasal spray targets the nasal cavity, which is the primary site for viral upper respiratory tract infections.

### Methods

This study was designed to measure the virucidal effectiveness of the disclosed formulations, and thereby determine the potential of the formulations for inactivation of the target virus - SARS-CoV-2 - in suspension.

The study followed the ASTM International test method designated E1052 "*Standard Test Method to Assess the Activity of Microbicides against Viruses in Suspension*". Severe Acute Respiratory Syndrome-Related Coronavirus 2 (SARS-CoV-2, COVID-19 virus), Strain: USA-WA1/2020, Source: BEI resources, NR-52281, was used. The virus stock was stored at an ultra-low temperature prior to use, and thawed on the day of the test. The challenge virus stock contained 5.0% serum. The composition of the tested formulations were prepared as described in Table 5. The test formulations were pre-equilibrated to the test temperature prior to the study. The test solutions were evaluated at one contact time at one replicate. For each run, an aliquot of 0.3 mL virus stock was added to a mix of 1.4 mL of the test solution + 1.3 mL of phosphate buffer and mixed by vortexing. The mixture was incubated at 35 - 37 °C for 30 minutes. After incubation, an aliquot or the entirety of the reaction mixture was pulled and immediately mixed with an equal volume of Newborn Calf Serum (NCS) and then vortexed. Selected dilutions were inoculated onto the host cells to assay for the quantity of infectious virus units.

### Control experiments

Multiple controls were carried out including a virus recovery control, neutralizer effectiveness/viral interference control, a cytotoxicity control, a media negative control and a virus stock titer control. The neutralizer effectiveness/viral interference control was performed in order to determine if residual active ingredients were present after neutralization and if the neutralized test substance interfered with virus infectivity. All controls were performed at the same time as the test, incubated under the same conditions and assayed in the same manner as the test.

### i. Virus recovery control:

A 2.7 mL aliquot of Minimum Essential Medium (MEM) + 2 % NCS was spiked with 0.3 mL of virus and mixed by vortexing. 30 minutes (the contact time) after virus was added, an aliquot or the entirety of the reaction mixture was mixed with an equal volume of a NCS via vortexing. Selected dilutions were inoculated onto the host cells to assay for the quantity of infectious virus, as described in the "infectivity Assay" section. The results from this control was used as the input viral load and compared with the test substance results to evaluate the viral reduction by the test substance.

### ii. Neutralizer effectiveness/viral interference control:

This control determined if residual active ingredient was present after neutralization and if the neutralized test substance interfered with virus infectivity. A 2.7-mL aliquot of the test substance (1.4 mL Test solution + 1.3 mL phosphate buffer) was spiked with 0.3 mL of Minimum Essential Medium (MEM) + 2 % NCS, mixed by vortexing and held for the contact time. Upon completion of the contact time, an aliquot or the entirety of the reaction mixture was immediately mixed with an equal volume of Minimum Essential Medium (MEM) + 2 % NCS medium via vortexing and was divided into two portions, one for cytotoxicity control and one for neutralizer effectiveness/viral interference control; and processed as the test.

### iii. Cytotoxicity control:

Selected dilutions of the sample obtained from the Neutralizer effectiveness/viral interference control test setup were inoculated onto host cells and incubated together with samples as described for the test procedure. The condition of the host cells were recorded at the end of the incubation period. The cytotoxic effects should be distinct from virus-specific cytopathic effects, which will be evident in the stock titer and virus recovery control cultures.

### iv. Cell viability control:

At least four wells were inoculated with an appropriate media during the incubation phase of the study. This control demonstrates if cells remain viable throughout the course of the assay period. In addition, it confirms if the sterility of the media employed throughout the assay period.

### v. Virus Stock Titer control:

An aliquot of the virus stock as used in the study was directly serially diluted and inoculated onto the host cells to confirm the titer of the stock virus.

This control demonstrates if the titer of the stock virus is appropriate for use and that the viral infectivity assay can be performed appropriately.

### Infectivity assay:

The residual infectious virus in the test and controls were detected by viral-induced cytopathic effect (CPE).

Selected dilutions of mixtures to be tested were added to cultured cell monolayers at a minimum of four wells per dilution per sample. The inoculated plates were incubated at 36±2°C in 5±3% CO₂ for 4-9 days. The host cells were washed twice with phosphate buffered saline prior to inoculation. The host cell cultures were observed and refed, as necessary, during the incubation period. The host cells were examined for presence of infectious virus following the completion of the incubation period. The resulting virus-specific CPE and test-article specific cytotoxic effects, when present, were scored by examining both test and controls. When necessary, virus was detected via staining with virus-specific antibody.

### Calculations:

The 50% tissue culture infective dose per mL (TCID50/mL) was determined using the method of Spearman-Karber (Kärber G. et al, 1931) or other appropriate methods such as Reed and Muench (Reed and Muench, 1938).

The Log10 Reduction Factor (LRF) was calculated in the following manner: Log10 Reduction Factor = Virus Recovery Control (Log10 TCID50) - Test (Log10 TCID50) where: Virus Load (Log10 TCID50) = Virus Titer (Log10 TCID50/mL) + Log10 [Volume (mL) x Volume correction (e.g., neutralization)].

### Results and conclusions

The formulation of TS01 and TS02 is presented in Table 5. Only the nasal spray contains carrageenan. The virucidal effects of throat spray (TS01) and nasal spray (TS02) are presented in 6.

**Table 5. Formulation of TS01 (Oral spray) and TS02 (Nasal spray) employed in the examples herein.**

| | **TS01 (wt%)** (Oral) | **TS02 (wt%)** (Nasal) |
|---|---|---|
| **Glycerol** | 55.2 | 29.6 |
| **Tris-HCl** | 0.12 | 0.12 |
| **CaCl₂** | 0.014 | 0.014 |
| **Water** | 39.4 | 45.6 |
| **Hyaluronic acid** | 0.00022 | 0.00022 |
| **Xylitol** | 4.9 | 24.4 |
| **Carrageenan** | - | 0.01 |
| **Spearmint** | 0.05 | - |
| **Trypsin** | 0.05 | 0.02 |
| **pH** | 7.5 | 6.5 |

The viral stock titer control for each assay confirmed that the appropriate titer was used in the experiment and sufficient amount of virus was recovered for the virus recovery control. Cytotoxicity was not detected at any dilution. All controls met the criteria for a valid test.

The data from the SARS-CoV-2 viral reduction test, is presented in 6. From the data it is clear, that both nasal and throat spray formulations reduces SARS-CoV-2 virus by >99.9% *in vitro.*

This shows, that targeting respectively the nose and the mouth with antimicrobial sprays, such as a nose spray or a mouth spray, is an efficient in preventing or reducing the possibility to be infected by viruses.

**Table 6. SARS-Co V-2 viral reduction.**

| **Formulation** | **Log reduction** | **Inactivation (%)** |
|---|---|---|
| TS01 (Throat spray) | 3.25 log₁₀ | 99.9 |
| TS02 (Nasal spray) | 3.12 log₁₀ | 99.9 |
| Commercially available, trypsin-comprising mouth spray | 1.76 log₁₀* | 98.3* |

| | | |
|---|---|---|
| **Gudmundsdottir et al. 2021.* | | |

### Example 6: Rheology experiments

The purpose of this experiment was to measure how viscosity changed upon adding carrageenan.

### Methods

Different formulations containing glycerol (55 wt%) and carrageenan (0-1 wt%) was mixed and the instantaneous viscosity of the solutions was measured. A throat spray solution TS03 (TS01 + 0.05 wt % carrageenan) was also evaluated.

### Rheology Measurements-Rotational Rheometer:

The rheological characteristics of the formulations were examined at high shear rates using continuous shear techniques and were performed with a controlled stress Malvern Rheometer (Malvern Instruments) using plate geometry radius 20 mm. The frequency sweep method was performed between 0.1 Hz and 10 Hz, with a shear strain of 0.8%, at 25 °C, while the table of shear rate method was performed by increasing the shear rate from 0.1 s⁻¹ to 100 s⁻¹, at 25 °C. The shear stress was measured by this method and the apparent viscosity was calculated by dividing the shear stress by the shear rate.

### Results and conclusions

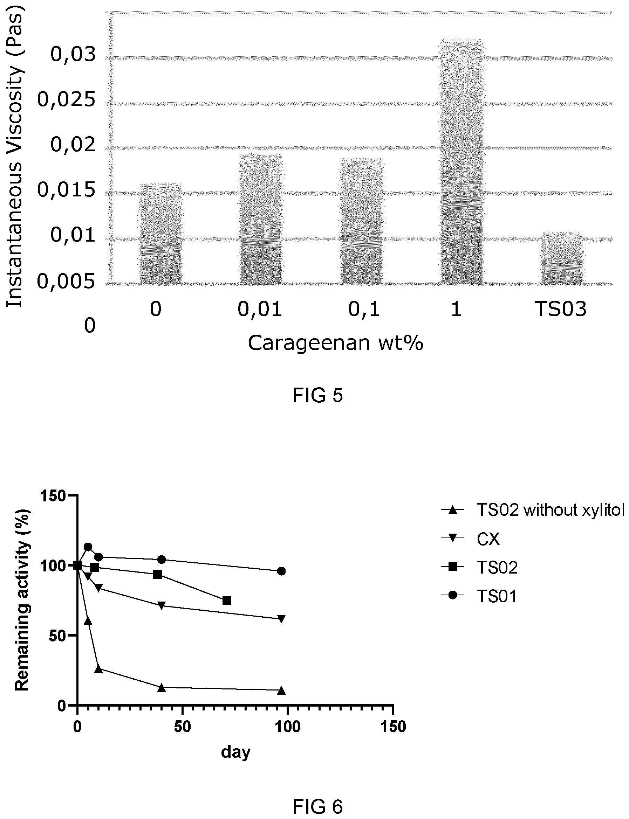

The results are presented in Figure 5. It was possible to add at least 0.1 wt% carrageenan without impacting viscosity. The addition of carrageenan to TS01 (providing TS03) did not increase the viscosity markedly, and thus the formulation can be used in a spray. The observed increase in viscosity indicates increased mucoadhesiveness.

### Example 7: Virucidal efficacy of throat spray against Influenza A virus

The purpose of the experiment was to determine virucidal efficacy of a throat spray formulation against Influenza A virus strain H3N2.

### Materials and methods

This study had the same experimental set up as thoroughly described in Example 5. This study also followed the ASTM International test method designated E1052 *"Standard Test Method to Assess the Activity of Microbicides against Viruses in Suspension*". The following specific changes to the experimental set up in Example 5 were made. Virus tested: Influenza A Virus strain, H3N2, Charles River Laboratories. Host cell line: MDCK cells, ATCC CCL-34. Dilution medium: Minimum Essential Medium (MEM) + 1.0 µg/ml Trypsin. Neutralizer: MEM + 1% Fetal Bovine Serum (FBS). Incubation time 6 days.

### Results and conclusion

The throat spray solution, Table 5, was tested for its ability to inactivate Influenza A Virus (H3N2), exposed to the TS01 throat spray solution in suspension for 30 minutes at 36°C. The viral stock titer control for the assay confirmed that the appropriate titer was used in the experiment and sufficient amount of virus was recovered for the virus recovery control. Cytotoxicity was not detected at any dilution. All controls met the criteria for a valid test.

The viral reductions for the TS01 solution against Influenza A Virus (H3N2) was a log reduction of 1.25 corresponding to 94.4% reduction. The TS01 solution is also effective against Influenza virus *in vitro.*

### Example 8: Cytopathic effect inhibition of respiratory syncytial virus (RSV)

To assess antiviral activity against RSV, cytopathic effect (CPE)-based inhibition assay was used in HEp-2 cells for T01 and T02, Table 5. CPE is morphological changes in cells caused by cytopathogenic virus infection.

### Materials and methods

### CPE inhibition assay

On day -1, HEp-2 cells were seeded in clear 96-well plates at 1.30E+04 cells per well. T01 and T02 were diluted 1:10 in PBS and added in triplicate to HEp-2 cells. Cells were incubated for 1-hour at 37°C . Approximately 200 PFU/well of RSV A2 (0.02 MOI) was then added to the TA/cells wells. Virus only and cells only were also included for controls and calculation, as well as an internal assay control was also included for assay validation. HEp-2 cells were incubated for 3 days at 37°C. On day 3 post infection cells were immuno-stained and optical density was read and the percentage of virus inhibition was determined.

### Cytotoxicity assay

T01 and T02 were diluted 1:10 in PBS for 3-days incubation with HEp-2 cells seeded in black 96-well plates; cells only and medium only wells were added. After 3 days cells were lysed for evaluation of the ATP content using Promega's Cell Titer Glo kit. The luciferase luminescence in relative light units (RLU) was read and the percentage of cytotoxicity was determined

### Results and Conclusion

Both T01 and T02 demonstrated inhibition against RSV but both solutions also demonstrated high toxicity against HEp-2 cells, Table 7. It is well known that high glycerol concentration is cytotoxic for human cells in general due to its high osmolality and therefore further dilutions could be performed to verify inhibition, at least for the T01 formulation.

**Table 7. RSVinhibition.**

| **Formulation** | **Cytotoxicity (%)** | **Inhibition (%)** |
|---|---|---|
| TS01 (Throat spray) | 91.6 | 100 |
| TS02 (Nasal spray) | 65.1 | 100 |

### Example 9: In vitro antiviral effect against SARS-CoV-2 (Omicron variant)

For this assay the Vesicular Stomatitis virus (VSV) glycoprotein (G) was substituted with SARS-CoV-2 B.1.1.529 (Omicron) spike (S) protein to create a recombinant virus that can be safely handled at biosafety level 2. In this example, T01 and T02 were incubated together with cells and then virus was added to infect cells. In Example 5, virus and T01 or T02 were pre-incubated and then added to cells.

### Materials and methods

### Luciferase-based inhibition assay

On day -1, Vero cells were seeded in black 96-well plates at 5.00E+04 cells per well. T01 and T02 were diluted 1:1 and 1:10 in PBS and added in triplicate to Vero cells. Cells were incubated for 1-hour at 37°C. Approximately 10,000 RLU of rVSV-SARS-CoV-2 B.1.1.529 Omicron was then added to the TA/cells wells. Virus only and cells only were also included for controls and calculation, as well as an internal assay control was also included for assay validation. Vero cells were incubated for 24-hours at 37°C. Firefly Luciferase activity was detected using the Bright-Glo^{™} Assay System kit (Promega) and the percentage of virus inhibition was determined.

### Cytotoxicity assay

T01 and T02 were diluted 1:1 and 1:10 in PBS for 24-hours incubation with Vero cells seeded in 96-well black plates; cells only and medium only wells were added. After 24-hours, cells were lysed for evaluation of the ATP content using Promega's Cell Titer Glo kit. The luciferase luminescence in relative light units (RLU) was read and the percentage of cytotoxicity was determined.

### Results and conclusion.

Low cytotoxicity was observed for both T01 and T02 at the 1:10 dilution. Both T01 and T02 demonstrated inhibition against SARS-CoV-2 B.1.1.529 at the 1:10 dilution formulation. High cytotoxicity was observed for both T01 and T02 at the 1:1 dilution. Table 8 shows the results.

**Table 8. SARS-CoV-2 B.1. 1.529 (Omicron) inhibition.**

| **Formulation** | **Dilution** | **Cytotoxicity (%)** | **Inhibition (%)** |
|---|---|---|---|
| TS01 (Throat spray) | 1:1 | 99 | 100 |
| | 1:10 | 15.5 | 92 |
| TS02 (Nasal spray) | 1:1 | 99 | 100 |
| | 1:10 | 22.9 | 97 |

### Example 10: Stability of a nasal and throat spray formulation

The purpose of the experiment was to measure enzyme stability in TS01 and TS02.

### Methods

TS01, TS02, TS02 without xylitol and a commercially available, trypsin-containing mouth spray (CX) were stored in sealed glass bottles. The stability of trypsin in each formulation composition were tested by placing them under stressed conditions (40 ± 2°C/100% RH) for up to 97 days. Enzyme activity was measured using same method as described in Example 1

### Results and conclusion

Results are presented in Figure 6. TS01 expressed the highest stability. Both TS01 and TS02 is more stable than CX. TS02 without xylitol is not stable

### References

Allan GM, Arroll B. Prevention and treatment of the common cold: making sense of the evidence. CMAJ. Can Med Assoc; 2014;186(3):190-9.
Craik CS et al. Proteases as therapeutics. Biochem J. 2011;435:1-16.
D'Souza et al, 2015
Gudmundsdottir, Ágústa, et al. Inactivation of SARS-CoV-2 and HCoV-229E in vitro by ColdZyme® a medical device mouth spray against the common cold. J Med Virol. 2021; 93: 1792- 1795.
Harkema JR, Carey SA, Wagner JG. The nose revisited: a brief review of the comparative structure, function, and toxicologic pathology of the nasal epithelium. Toxicologic pathology. Sage Publications; 2006;34(3):252-69.
Heikkinen T, Järvinen A. The common cold. Lancet. 2003 Jan 3;361(9351):51-9.
Jackson GG., Dowling HF, Spiesman IG, Boand AV.. Transmission of the common cold to volunteers under controlled conditions. I. The common cold as a clinical entity. AMA Arch Intern Med. 1958 Feb;101(2):267-78.
Johnston SL et al. The relationship between upper respiratory infections and hospital admissions for asthma: a time-trend analysis. Am J Respir Crit Care Med. American Public Health Association; 1996;154(3):654-60.
Karber, G. Beitrag zur kollecktiven Behandlung pharmakologischer Reihenversuche. Arch. Exptl. Pathol. Pharmakol, 162, 480-483, 1931.
Lenoir J, Adriaens E, Remon JP. New aspects of the Slug Mucosal Irritation assay: predicting nasal stinging, itching and burning sensations. Journal of Applied Toxicology. Wiley Online Library; 2011;31(7):640-8.
Mallia, Patrick, and Sebastian L. Johnston. Influenza infection and COPD.International journal of chronic obstructive pulmonary disease; 2007:2(1): 55-64.
Meena et al, A Review on Microbial Pathogenesis and Host Response. Model Organisms for Microbial Pathogenesis, Biofilm Formation and Antimicrobial Drug Discovery. Springer, Singapore, 2020. 47-60.
Pappas DE, Hendley JO, Hayden FG, Winther B. Symptom profile of common colds in school-aged children. The Pediatric infectious disease journal. LWW; 2008;27(1):8-11.
Posch W, Vosper J, Zaderer V, Noureen A, Constant S, Bellmann-Weiler R, et al. ColdZyme Maintains Integrity in SARS-CoV-2-lnfected Airway Epithelia. Mbio. Am Soc Microbiol; 2021;12(2).
Ramalingam S, Graham C, Dove J, Morrice L, Sheikh A. A pilot, open labelled, randomised controlled trial of hypertonic saline nasal irrigation and gargling for the common cold. Scientific reports. Nature Publishing Group; 2019;9(1):1-11.
Reed L.J and Muench H. A simple method of estimating fifty percent endpoints. American Journal of Epidemiology, Volume 27, Issue 3, May 1938, Pages 493-497.
Salade L, Wauthoz N, Goole J, Amighi K. How to characterize a nasal product. The state of the art of in vitro and ex vivo specific methods. Int J Pharm. Elsevier; 2019;561:47-65.
Sungnak W, Huang N, Bécavin C, Berg M, Queen R, Litvinukova M, et al. SARS-CoV-2 entry factors are highly expressed in nasal epithelial cells together with innate immune genes. Nature medicine. Nature Publishing Group; 2020;26(5):681-7.
Trangsrud AJ, Whitaker AL, Small RE. Intranasal corticosteroids for allergic rhinitis. Pharmacotherapy: The Journal of Human Pharmacology and Drug Therapy. Wiley Online Library; 2002;22(11):1458-67.
Zelikin AN, Stellacci F. Broad-Spectrum Antiviral Agents Based on Multivalent Inhibitors of Viral Infectivity. Advanced Healthcare Materials. Wiley Online Library; 2021;10(6):2001433.

## Claims

1. A composition comprising:
i. 0.005 to 1.0 % w/w trypsin;
ii. 20 to 70 % w/w glycerol; and
iii. 1.0 to 65 % w/w xylitol.

2. The composition according to claim 1, wherein the composition further comprises 0.003 to 0.2 % w/w salt comprising a divalent cation, such as 0.003 to 0.15 % w/w, such as 0.003 to 0.1 % w/w salt comprising a divalent cation, such as wherein the salt comprising a divalent cation is a pharmaceutically acceptable salt of calcium, including hydrates thereof, such as wherein the salt comprising a divalent cation is CaCl₂.

3. The composition according to any one of the preceding claims,
wherein the composition comprises 25 to 65 % w/w glycerol, such as 27 to 57 % w/w glycerol;
wherein the composition comprises 3.0 to 50 % w/w xylitol, such as 4.5 to 30 % w/w xylitol, and/or
wherein the combined amount of glycerol and xylitol is at least 40 % w/w, such as at least 45 % w/w, such as at least 50 % w/w.

4. The composition according to any one of the preceding claims:
wherein the composition further comprises a buffer, such as wherein the concentration of the buffer is 1 to 200 mM, such as wherein the buffer is selected from the group consisting of tris(hydroxymethyl)amino methane (Tris), 3-(*N*-morpholino)propanesulfonic acid (MOPS) or phosphate; and/or
wherein the pH of the composition is 5.5 to 8.5.

5. The composition according to any one of the preceding claims, wherein the composition further comprises water, such as at most 50 % w/w water, such as at most 40 % w/w, such as at most 30 % w/w.

6. The composition according to any one of the preceding claims, wherein the composition comprises or consists essentially of:
i. 0.005 to 1.0 % w/w trypsin;
ii. 20 to 70 % w/w glycerol;
iii. 1.0 to 65 % w/w xylitol;
iv. 0.0001 to 1.5 % w/w hyaluronic acid;
v. 0.002 to 0.2 % w/w CaCl₂; and
vi. 0.01 to 1.2 % w/w buffer
such as wherein the composition comprises or consists essentially of:
i. 0.015 to 0.055 % w/w trypsin;
ii. 27 to 57 % w/w glycerol;
iii. 4.5 to 30 % w/w xylitol;
iv. 0.0002 to 0.02 % w/w hyaluronic acid;
v. 0.010 to 0.015 % w/w CaCl₂; and
vi. 0.1 to 0.14 % w/w buffer.

7. The composition according to any one of the preceding claims, further comprising carrageenan such as wherein the composition comprises no more than 1 % w/w carrageenan, or further comprising a flavouring agent such as spearmint or eucalyptus.

8. The composition according to any one of the preceding claims, wherein the composition is in the form of a spray such as a nasal spray or a throat spray, lozenge, pastille, chewing gum, gel or liquid.

9. The composition according to any one of the preceding claims for use in medicine.

10. The composition according to any one of claims 1 to 8 for use in the prevention and/or treatment of a disease, disorder or condition selected from the group consisting of microbial infections, such as a microbial infection selected from the group consisting of viral infections, bacterial infections, fungal infections, and yeast infections; and oral conditions, such as gingivitis or gum disease in and/or on a mammal.

11. The composition for use according to claim 10, wherein the viral infection is an upper respiratory tract viral infection, such as wherein the upper respiratory tract viral infection results in common cold, flu, rhinitis, sinusitis, COVID-19 such as the omicron variant, or a disease caused by upper respiratory tract viral infections.

12. The composition for use according to claim 10, wherein the viral infection is from a virus selected from the group consisting of rhinovirus, influenza virus such as influenza A virus, Respiratory Syncytial Virus (RSV), coronavirus, parainfluenza virus, adenovirus, enterovirus, metapneumovirus, and other infectious viruses,
or wherein the viral infection cause infectious diseases such as common cold, flu, rhinitis, sinusitis, bronchitis, Severe acute respiratory syndrome (SARS), Middle East respiratory syndrome (MERS), coronavirus disease 2019 (COVID-19) such as the COVID-19 omicron variant, Pneumonia, Viral Meningitis, Herpangina, herpesvirus, papillomavirus or any other disease caused by viral infections.

13. The composition for use according to any one of claims 10 to 12, wherein the prevention and/or treatment is the prevention and/or treatment of symptoms related to viral infection, such as sore throat, malaise, rhinorrhea, nasal congestion, headache, cough, sneezing, and/or fever.

14. The composition for use according to any one of claims 10 to 13, wherein the composition is for nasal or oral use.

15. A method of manufacturing the composition according to any one of claims 1 to 8 comprising mixing;
i. trypsin;
ii. glycerol; and
iii. xylitol; and optionally any of the further ingredients according to claims 2-8.

## Patentansprüche

1. Zusammensetzung, umfassend:
i. 0,005 bis 1,0 Gew.-% Trypsin;
ii. 20 bis 70 Gew.-% Glycerin; und
iii. 1,0 bis 65 Gew.-% Xylitol.

2. Zusammensetzung nach Anspruch 1, wobei die Zusammensetzung ferner 0,003 bis 0,2 Gew.-% Salz umfasst, das ein zweiwertiges Kation umfasst, wie 0,003 bis 0,15 Gew.-%, wie 0,003 bis 0,1 Gew.-% Salz, das ein zweiwertiges Kation umfasst, wobei das Salz, das ein zweiwertiges Kation umfasst, ein pharmazeutisch verträgliches Salz von Calcium, einschließlich Hydraten davon, ist, wobei das Salz, das ein zweiwertiges Kation umfasst, CaCl₂ ist.

3. Zusammensetzung nach einem der vorhergehenden Ansprüche,
wobei die Zusammensetzung 25 bis 65 Gew.-% Glycerin, wie 27 bis 57 Gew.-% Glycerin, umfasst;
wobei die Zusammensetzung 3,0 bis 50 Gew.-% Xylitol, wie 4,5 bis 30 Gew.-% Xylitol umfasst, und/oder
wobei die kombinierte Menge an Glycerin und Xylitol mindestens 40 Gew.-%, wie mindestens 45 Gew.-%, wie mindestens 50 Gew.-% beträgt.

4. Zusammensetzung nach einem der vorhergehenden Ansprüche:
wobei die Zusammensetzung ferner einen Puffer umfasst, wobei die Konzentration des Puffers 1 bis 200 mM beträgt, wobei der Puffer ausgewählt ist aus der Gruppe bestehend aus Tris(hydroxymethyl)amino-methan (Tris), 3-(*N-*Morpholino)propansulfonsäure (MOPS) oder Phosphat; und/oder wobei der pH-Wert der Zusammensetzung 5,5 bis 8,5 beträgt.

5. Zusammensetzung nach einem der vorhergehenden Ansprüche, wobei die Zusammensetzung ferner Wasser umfasst, wie höchstens 50 Gew.-% Wasser, wie höchstens 40 Gew.-%, wie höchstens 30 Gew.-%.

6. Zusammensetzung nach einem der vorhergehenden Ansprüche, wobei die Zusammensetzung Folgendes umfasst oder im Wesentlichen aus Folgendem besteht:
i. 0,005 bis 1,0 Gew.-% Trypsin;
ii. 20 bis 70 Gew.-% Glycerin;
iii. 1,0 bis 65 Gew.-% Xylitol;
iv. 0,0001 bis 1,5 Gew.-% Hyaluronsäure;
v. 0,002 bis 0,2 Gew.-% CaCl₂; und
vi. 0,01 bis 1,2 Gew.-% Puffer
wobei die Zusammensetzung Folgendes umfasst oder im Wesentlichen aus Folgendem besteht:
i. 0,015 bis 0,055 Gew.-% Trypsin;
ii. 27 bis 57 Gew.-% Glycerin;
iii. 4,5 bis 30 Gew.-% Xylitol;
iv. 0,0002 bis 0,02 Gew.-% Hyaluronsäure;
v. 0,010 bis 0,015 Gew.-% CaCl₂; und
vi. 0,1 bis 0,14 Gew.-% Puffer.

7. Zusammensetzung nach einem der vorhergehenden Ansprüche, ferner umfassend Carrageenan, wobei die Zusammensetzung nicht mehr als 1 Gew.-% Carrageenan umfasst, oder ferner umfassend einen Aromastoff wie Minze oder Eukalyptus.

8. Zusammensetzung nach einem der vorhergehenden Ansprüche, wobei die Zusammensetzung in Form eines Sprays wie Nasenspray oder Rachenspray, Lutschtablette, Pastille, Kaugummi, Gel oder Flüssigkeit vorliegt.

9. Zusammensetzung nach einem der vorhergehenden Ansprüche zur Verwendung in der Medizin.

10. Zusammensetzung nach einem der Ansprüche 1 bis 8 zur Verwendung bei der Vorbeugung und/oder Behandlung einer Krankheit, Störung oder eines Zustands, ausgewählt aus der Gruppe bestehend aus mikrobiellen Infektionen, wie einer mikrobiellen Infektion, ausgewählt aus der Gruppe bestehend aus viralen Infektionen, bakteriellen Infektionen, Pilzinfektionen und Hefeinfektionen, und oralen Zuständen, wie Gingivitis oder Zahnfleischerkrankungen in und/oder an einem Säugetier.

11. Zusammensetzung zur Verwendung nach Anspruch 10, wobei die Virusinfektion eine Virusinfektion der oberen Atemwege ist, wobei die Virusinfektion der oberen Atemwege zu einer Erkältung, Grippe, Rhinitis, Sinusitis, COVID-19, wie der Omicron-Variante, oder einer durch Virusinfektionen der oberen Atemwege verursachten Krankheit führt.

12. Zusammensetzung zur Verwendung nach Anspruch 10, wobei die Virusinfektion von einem Virus stammt, das ausgewählt ist aus der Gruppe bestehend aus Rhinovirus, Influenzavirus wie Influenza-A-Virus, Atemwegs-Synzytialvirus (RSV), Coronavirus, Parainfluenzavirus, Adenovirus, Enterovirus, Metapneumovirus und anderen infektiösen Viren,
oder wobei die Virusinfektion Infektionskrankheiten wie Erkältung, Grippe, Rhinitis, Sinusitis, Bronchitis, Schweres Akutes Atemwegssyndrom (SARS), Nahost-Atemwegssyndrom (MERS), Coronavirus-Krankheit 2019 (COVID-19) wie die COVID-19 Omicron-Variante, Lungenentzündung, virale Meningitis, Herpangina, Herpesvirus, Papillomavirus oder jede andere durch Virusinfektionen verursachte Krankheit verursacht.

13. Zusammensetzung zur Verwendung nach einem der Ansprüche 10 bis 12, wobei es sich bei der Vorbeugung und/oder Behandlung um die Vorbeugung und/oder Behandlung von Symptomen handelt, die eine Virusinfektion betreffen, wie Halsschmerzen, Unwohlsein, Schnupfen, nasale Kongestion, Kopfschmerzen, Husten, Niesen und/oder Fieber.

14. Zusammensetzung zur Verwendung nach einem der Ansprüche 10 bis 13, wobei die Zusammensetzung zur nasalen oder oralen Verwendung bestimmt ist.

15. Verfahren zum Herstellen der Zusammensetzung nach einem der Ansprüche 1 bis 8, umfassend Mischen von;
i. Trypsin;
ii. Glycerin und
iii. Xylitol; und gegebenenfalls einem der weiteren Bestandteile nach den Ansprüchen 2 bis 8.

## Revendications

1. Composition comprenant :
i. 0,005 à 1,0 % p/p de trypsine ;
ii. 20 à 70 % p/p de glycérol ; et
iii. 1,0 à 65 % p/p de xylitol.

2. Composition selon la revendication 1, dans laquelle la composition comprend également 0,003 à 0,2 % p/p de sel comprenant un cation divalent, par exemple 0,003 à 0,15 % p/p, par exemple 0,003 à 0,1 % p/p de sel comprenant un cation divalent, par exemple dans laquelle le sel comprenant un cation divalent est un sel pharmaceutiquement acceptable de calcium, y compris ses hydrates, par exemple dans laquelle le sel comprenant un cation divalent est CaCl₂.

3. Composition selon l'une quelconque des revendications précédentes,
dans laquelle la composition comprend 25 à 65 % p/p de glycérol, par exemple 27 à 57 % p/p de glycérol ;
dans laquelle la composition comprend 3,0 à 50 % p/p de xylitol, par exemple 4,5 à 30 % p/p de xylitol, et/ou
dans laquelle la quantité combinée de glycérol et de xylitol est d'au moins 40 % p/p, par exemple d'au moins 45 % p/p, par exemple d'au moins 50 % p/p.

4. Composition selon l'une quelconque des revendications précédentes :
dans laquelle la composition comprend également un tampon, par exemple dans laquelle la concentration du tampon est de 1 à 200 mM, par exemple dans laquelle le tampon est choisi dans le groupe constitué du tris(hydroxyméthyl)amino méthane (Tris), de l'acide 3-(N-morpholino)propanesulfonique (MOPS) ou du phosphate ; et/ou
dans laquelle le pH de la composition est de 5,5 à 8,5.

5. Composition selon l'une quelconque des revendications précédentes, dans laquelle la composition comprend également de l'eau, par exemple au plus 50 % p/p d'eau, par exemple au plus 40 % p/p, par exemple au plus 30 % p/p.

6. Composition selon l'une quelconque des revendications précédentes, dans laquelle la composition comprend ou est composée essentiellement de :
i. 0,005 à 1,0 % p/p de trypsine ;
ii. 20 à 70 % p/p de glycérol ;
iii. 1,0 à 65 % p/p de xylitol ;
iv. 0,0001 à 1,5 % p/p d'acide hyaluronique ;
v. 0,002 à 0,2 % p/p de CaCl₂ ; et
vi. 0,01 à 1,2 % p/p de tampon
par exemple dans laquelle la composition comprend ou est composée essentiellement de :
i. 0,015 à 0,055 % p/p de trypsine ;
ii. 27 à 57 % p/p de glycérol ;
iii. 4,5 à 30 % p/p de xylitol ;
iv. 0,0002 à 0,02 % p/p d'acide hyaluronique ;
v. 0,010 à 0,015 % p/p de CaCl₂ ; et
vi. 0,1 à 0,14 % p/p de tampon.

7. Composition selon l'une quelconque des revendications précédentes, comprenant également du carraghénane, par exemple dans laquelle la composition ne comprend pas plus de 1 % p/p de carraghénane, ou comprenant également un agent aromatisant par exemple la menthe verte ou l'eucalyptus.

8. Composition selon l'une quelconque des revendications précédentes, dans laquelle la composition se présente sous la forme d'un spray par exemple un spray nasal ou un spray pour la gorge, une tablette, une pastille, une gomme à mâcher, un gel ou un liquide.

9. Composition selon l'une quelconque des revendications précédentes pour une utilisation en médecine.

10. Composition selon l'une quelconque des revendications 1 à 8, pour une utilisation dans la prévention et/ou le traitement d'une maladie, d'un trouble ou d'une affection choisi(e) dans le groupe constitué d'infections microbiennes, par exemple une infection microbienne choisie dans le groupe constitué d'infections virales, d'infections bactériennes, d'infections fongiques et d'infections à levures ; et d'affections buccales, par exemple la gingivite ou une maladie des gencives chez et/ou sur un mammifère.

11. Composition pour une utilisation selon la revendication 10, dans laquelle l'infection virale est une infection virale des voies respiratoires supérieures, par exemple dans laquelle l'infection virale des voies respiratoires supérieures entraîne un rhume, une grippe, une rhinite, une sinusite, la COVID-19 par exemple le variant omicron, ou une maladie provoquée par des infections virales des voies respiratoires supérieures.

12. Composition pour une utilisation selon la revendication 10, dans laquelle l'infection virale provient d'un virus choisi dans le groupe constitué d'un rhinovirus, du virus de la grippe par exemple le virus de la grippe A, le virus respiratoire syncytial (VRS), un coronavirus, un virus para-influenza, un adénovirus, un entérovirus, un métapneumovirus et d'autres virus infectieux,
ou dans laquelle l'infection virale provoque des maladies infectieuses par exemple le rhume, la grippe, la rhinite, la sinusite, la bronchite, le syndrome respiratoire aigu sévère (SRAS), le syndrome respiratoire du Moyen-Orient (MERS), la maladie à coronavirus 2019 (COVID-19) par exemple le variant omicron de la COVID-19, la pneumonie, la méningite virale, l'herpangine, l'herpèsvirus, le papillomavirus ou toute autre maladie causée par des infections virales.

13. Composition pour une utilisation selon l'une quelconque des revendications 10 et 12, dans laquelle la prévention et/ou le traitement est la prévention et/ou le traitement de symptômes liés à une infection virale, par exemple maux de gorge, malaise, rhinorrhée, congestion nasale, maux de tête, toux, éternuements et/ou fièvre.

14. Composition pour une utilisation selon l'une quelconque des revendications 10 à 13, dans laquelle la composition est destinée à une utilisation nasale ou orale.

15. Procédé de fabrication de la composition selon l'une quelconque des revendications 1 à 8 comprenant le mélange de;
i. trypsine ;
ii. glycérol ; et
iii. xylitol ; et éventuellement l'un quelconque des autres ingrédients selon les revendications 2 à 8.
